# EUROPÄISCHE PATENTANMELDUNG

(11) **EP 4 403 562 A1**
(43) Veröffentlichungstag der Anmeldung: **24.07.2024**
(21) Anmeldenummer: 23152172.5
(22) Anmeldetag: 18.01.2023
(51) Int. Cl.: C07F 9/6574

(54) **DIPHOSPHITE MIT TERT-BUTYLRESTEN UND METHYLRESTEN**

(71) Anmelder: Evonik Operations GmbH, 45128 Essen (DE)
(72) Erfinder: SALE, Anna Chiara, 45657 Recklinghausen (DE); FRANKE, Robert, 45772 Marl (DE); BRÄCHER, Alexander, 45721 Haltern am See (DE); FRIDAG, Dirk, 45721 Haltern am See (DE); KNOSSALLA, Johannes, 46514 Gahlen (DE); KUCMIERCZYK, Peter, 44628 Herne (DE); MARKOVIC, Ana, 45721 Haltern am See (DE)
(74) Vertreter: Evonik Patent Association

(57) **Zusammenfassung**

Diphosphite mit tert-Butylresten und Methylresten und deren Verwendung in der Hydroformylierung.

## Beschreibung

Die Erfindung betrifft Diphosphite mit tert-Butylresten und Methylresten und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012 (112), 11, 5675-5732, DOI:10.1021/cr3001803.

Die technische Aufgabe der Erfindung ist die Bereitstellung einer Verbindung, mit welcher bei der Hydroformylierung von Olefinen eine gesteigerte Ausbeute erzielt werden kann.

Die Aufgabe wird gelöst durch eine Verbindung gemäß Anspruch 1.

Verbindung gemäß Formel **(I):** 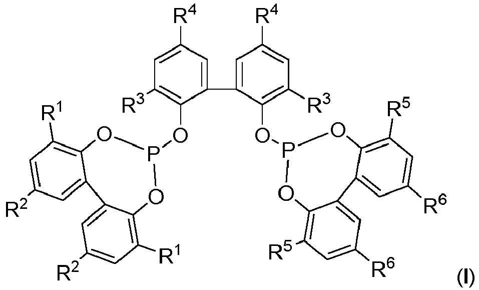
wobei R¹, R², R³, R⁴, R⁵, R⁶ für -CH₃ oder -*^{tert}*Bu stehen, und
mindestens einer der Reste R², R⁴, R⁶ für -*^{tert}*Bu steht, und
mindestens einer der Reste R¹, R⁵ für -CH₃ steht.

In einer Ausführungsform stehen R³ und R⁴ für den gleichen Rest.

In einer Ausführungsform stehen R¹ und R² für unterschiedliche Reste.

In einer Ausführungsform stehen R¹ und R² und auch R⁵ und R⁶ jeweils paarweise für unterschiedliche Reste.

R¹ steht also für einen anderen Rest als R² und gleichzeitig steht R⁵ für einen anderen Rest als R⁶.

In einer Ausführungsform stehen R² und R⁶ für den gleichen Rest.

In einer Ausführungsform stehen R² und R⁴ für den gleichen Rest.

In einer Ausführungsform stehen R² und R³ für den gleichen Rest.

In einer Ausführungsform stehen R², R⁶ für -*^{tert}*Bu.

In einer Ausführungsform steht R⁴ für -*^{tert}*Bu.

In einer Ausführungsform stehen R², R⁴, R⁶für -*^{tert}*Bu.

In einer Ausführungsform steht R³ für -*^{tert}*Bu.

In einer Ausführungsform stehen R¹, R⁵ für -CH₃.

In einer Ausführungsform weist Verbindung die Struktur **(1)** auf:

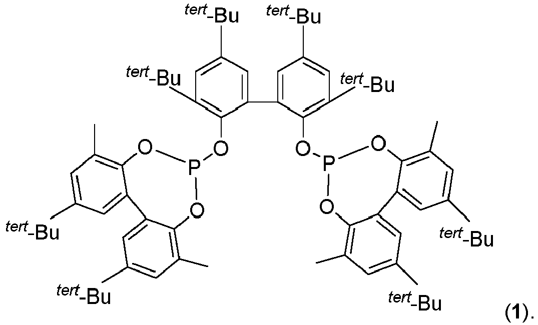

Im Folgenden soll die Erfindung anhand eines Ausführungsbeispieles näher erläutert werden.

### Katalyseversuche

Die Hydroformylierung wurde in einem mit Druckkonstanthaltung, Gasflussmessung, und Begasungsrührer ausgestatteten 16 ml-Autoklaven der HEL group, Hertfordshire, Großbritannien, durchgeführt. Das Substrat (1-Octen) wurde mehrere Stunden über Natrium am Rückfluss erhitzt und unter Argon destilliert.

Für die Versuche wurden die Reaktionslösungen vorab unter Argonatmosphäre vorbereitet. Hierfür wurden 100 ppm Rh(acac)(CO)₂ und die entsprechende Menge an Phosphitverbindung (L:Rh = 50:1) eingewogen und mit 8,0 ml Toluol aufgefüllt. Die jeweils eingebrachte Masse an Toluol wurde für die GC-Analyse bestimmt. Anschließend wurden 1,80 g 1-Octen (16 mmol) hinzugegeben. Die vorbereiteten Lösungen wurden anschließend in den Autoklaven eingefüllt und dieser dreimal mit Argon und dreimal mit Synthesegas (Linde; H₂ (99,999 %) : CO (99,997%) = 1:1) gespült. Dann wurde der Autoklav bei einem Gesamtdruck von 10 bar unter Rühren (900 U/min) auf die angestrebte Temperatur aufgeheizt. Nach Erreichen der Reaktionstemperatur wurde der Synthesegasdruck auf 17 bar erhöht und die Reaktion bei konstantem Druck für 1 h geführt. Der Autoklav wurde nach Ablauf der Reaktionszeit auf Raumtemperatur abgekühlt, unter Rühren entspannt und mit Argon gespült. Jeweils 0,5 ml der Reaktionsmischungen wurden nach Beenden der Reaktion entnommen, mit 4 ml Pentan verdünnt und gaschromatographisch analysiert: HP 5890 Series II plus, PONA, 50 m × 0,2 mm × 0,5 µm. Die quantitative Bestimmung von Restolefin und Aldehyd erfolgte gegen das Lösungsmittel Toluol als interner Standard.

Der Versuch wurde mit den Verbindungen **(1)** und **(2)** durchgeführt.

Die Verbindung **(2)** dient hierbei als Vergleichsligand.

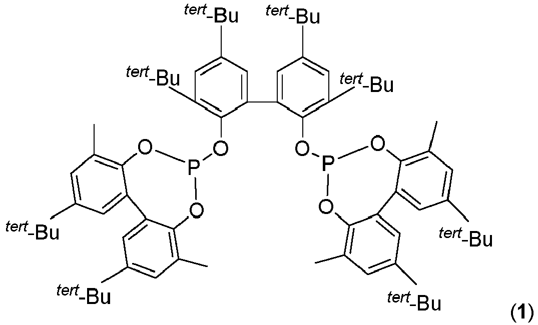

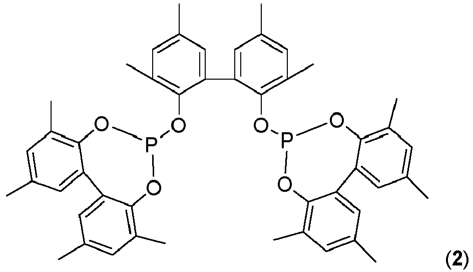

### Ergebnisse der Katalyseversuche

[Rh]: 100 ppm, p: 17 bar, T: 120 °C; t: 1 h

**Tabelle: Hydroformylierung von 1-Octen**

| Ligand | Ausbeute [%] |
|---|---|
| **(1)*** | 26 |
| **(2)** | 11 |

| | |
|---|---|
| * erfindungsgemäße Verbindung | |

Die durchgeführten Versuche belegen, dass die gestellte Aufgabe durch eine erfindungsgemäße Verbindung gelöst wird.

## Patentansprüche

1. Verbindung gemäß Formel (I): 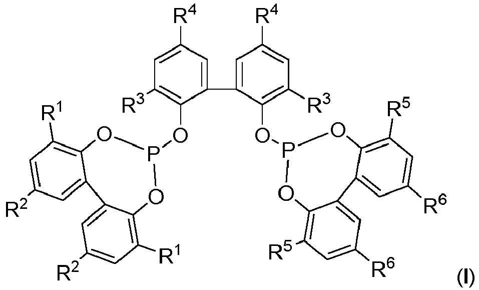
wobei R¹, R², R³, R⁴, R⁵, R⁶ für -CH₃ oder -*^{tert}*Bu stehen, und
mindestens einer der Reste R², R⁴, R⁶ für -*^{tert}*Bu steht, und
mindestens einer der Reste R¹, R⁵ für -CH₃ steht.

2. Verbindung nach Anspruch 1,
wobei R³ und R⁴ für den gleichen Rest stehen.

3. Verbindung nach einem der Ansprüche 1 oder 2,
wobei R¹ und R² für unterschiedliche Reste stehen.

4. Verbindung nach einem der Ansprüche 1 bis 3,
wobei R¹ und R² und auch R⁵ und R⁶ jeweils paarweise für unterschiedliche Reste stehen.

5. Verbindung nach einem der Ansprüche 1 bis 4,
wobei R² und R⁶ für den gleichen Rest stehen.

6. Verbindung nach einem der Ansprüche 1 bis 5,
wobei R² und R⁴ für den gleichen Rest stehen.

7. Verbindung nach einem der Ansprüche 1 bis 6,
wobei R² und R³ für den gleichen Rest stehen.

8. Verbindung nach einem der Ansprüche 1 bis 7,
wobei R², R⁶ für -*^{tert}*Bu stehen.

9. Verbindung nach einem der Ansprüche 1 bis 8,
wobei R⁴ für -*^{tert}*Bu steht.

10. Verbindung nach einem der Ansprüche 1 bis 9,
wobei R², R⁴, R⁶ für -*^{tert}*Bu stehen.

11. Verbindung nach einem der Ansprüche 1 bis 10,
wobei R³ für -*^{tert}*Bu steht.

12. Verbindung nach einem der Ansprüche 1 bis 11,
wobei R¹, R⁵ für -CH₃ stehen.

13. Verbindung nach einem der Ansprüche 1 bis 12,
die Verbindung die Struktur **(1)** aufweist:
